(19) Europäisches Patentamt
European Patent Office
Office européen des brevets

(11) Veröffentlichungsnummer : **0 087 701**
**B1**

# (12) EUROPÄISCHE PATENTSCHRIFT

(45) Veröffentlichungstag der Patentschrift : **20.08.86**

(51) Int. Cl.⁴ : **C 07 D263/58**

(21) Anmeldenummer : **83101589.6**

(22) Anmeldetag : **19.02.83**

## (54) Verfahren zur Herstellung von 2-Chlorbenzoxazolen.

(30) Priorität : **27.02.82 DE 3207153**

(43) Veröffentlichungstag der Anmeldung : **07.09.83 Patentblatt 83/36**

(45) Bekanntmachung des Hinweises auf die Patenterteilung : **20.08.86 Patentblatt 86/34**

(84) Benannte Vertragsstaaten : **CH DE FR GB IT LI NL**

(56) Entgegenhaltungen :
**DE-B- 1 164 413**
**JOURNAL OF ORGANIC CHEMISTRY, Band 23, Nr. 10, 30. Oktober 1958, Seiten 1500-1503, Washington, D.C.; J. SAM et al.: "Methylated 2-amino-5-chlorobenzoxazoles"**

(73) Patentinhaber : **CASSELLA Aktiengesellschaft**
**Hanauer Landstrasse 526**
**D-6000 Frankfurt am Main 61 (DE)**

(72) Erfinder : **Becherer, Johannes, Dr.**
**Bahnhofstrasse 143a**
**D-6457 Maintal-Hochstadt (DE)**
Erfinder : **Kühlein, Klaus, Dr.**
**Fasanenstrasse 41**
**D-6233 Kelkheim/Ts. (DE)**
Erfinder : **Kussmaul, Ulrich, Dr.**
**Tannenweg 39A**
**D-6367 Karben 1 (DE)**

(74) Vertreter : **Urbach, Hans-Georg, Dr. et al**
**Hanauer Landstrasse 526**
**D-6000 Frankfurt am Main 61 (DE)**

**EP 0 087 701 B1**

Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Jouve, 18, rue St-Denis, 75001 Paris, France

**Beschreibung**

Die Erfindung betrifft ein Verfahren zur Herstellung von 2-Chlorbenzoxazolen der Formel I

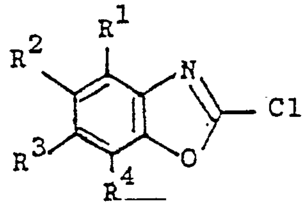

(I)

worin $R^1$, $R^2$, $R^3$, $R^4$ unabhängig voneinander H, Cl oder Alkyl mit 1 bis 4 C-Atomen bedeuten, durch Umsetzung von 2-Mercaptobenzoxazolen der Formel II

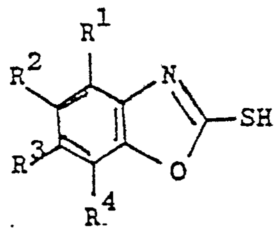

(II)

mit Chlor.

Die Herstellung von 2-Chlorbenzoxazol aus 2-Mercaptobenzoxazol und Chlor oder chlorabspaltenden Mitteln ist bekannt, vgl. J.pr. Chemie *42*, 454 (1890) ; Am. Chemical Journal *21*, 123 (1899) ; Boll. Sci. Fac. Chim. Ind. Bologna *23* (2-3) 89-98 (1965) ; DE-PS 11 64 413 ; DE-OS 12 10 617. Alle bekannten Verfahren besitzen Nachteile. Bei den Verfahren, bei denen eine große Menge eines inerten Lösungs- oder Suspendierungsmittels verwendet wird, bestehen diese Nachteile insbesondere in der schlechten Raumausbeute und den dadurch bedingten hohen Investitionskosten, in dem hohen Energiebedarf für das Abdestillieren des Lösungsmittels und in der schwierigen Auftrennung des Reaktionsgemisches in seine Bestandteile. So ist es z. B. schwierig, die bei der Chlorierung mit Chlor entstehenden Schwefelchloride von niedrigsiedenden Lösungsmitteln abzutrennen, andererseits ist bei Verwendung höhersiedender Lösungsmittel die Trennung von Lösungsmittel und Produkt aufwendig.

Es sind lediglich zwei Verfahren beschrieben, die keine Lösungsmittel verwenden. Bei dem einen (J.pr. Chemie *42*, 454 (1980)) tritt stets eine teilweise Verkohlung auf, was eine beträchtliche Ausbeuteverringerung zur Folge hat. Bei dem anderen (Boll. Sci. Fac. Chim. Ind. Bologna *23* (2-3) 89-98 (1965)) wird bei sehr aufwendiger Aufarbeitung keine Ausbeute angegeben. Wird destillativ aufgearbeitet, beträgt die Ausbeute nur 85 % der Theorie. Außerdem verbleibt eine große Menge Destillationsrückstand, dessen Vernichtung bei Herstellung im größeren Maßstab Probleme aufwirft.

Es wurde nun überraschenderweise gefunden, daß die Herstellung von 2-Chlorbenzoxazolen der Formel I durch Umsetzung von 2-Mercaptobenzoxazolen der Formel II mit Chlor ohne Verwendung eines zusätzlichen Lösungsmittels oder Katalysators in hoher Ausbeute und Reinheit möglich ist, wenn in ein geschmolzenes 2-Chlorbenzoxazol der Formel I 2-Mercaptobenzoxazol der Formel II, wobei die beiden Verbindungen in den Resten $R^1$ bis $R^4$ gleich substituiert sind, eingetragen und anschließend oder gleichzeitig Chlor eingeleitet wird und daß, bezogen auf die Gesamtmenge des eingetragenen 2-Mercaptobenzoxazols, die Menge des geschmolzenen 2-Chlorbenzoxazols mindestens 8 Gew.% beträgt.

Bei der Durchführung des erfindungsgzemäßen Verfahrens legt man eine geringe Menge eines geschmolzenen 2-Chlorbenzoxazols der Formel I vor, trägt unter Rühren 2-Mercaptobenzoxazol der Formel II ein und leitet Chlor ein. Bezogen auf die Gesamtmenge des zum Einsatz kommenden 2-Mercaptobenzoxazols der Formel II werden z. B. 8 bis 30 Gew.%, vorzugsweise 10 bis 25 Gew.%, 2-Chlorbenzoxazol der Formel I vorgelegt. Eine größere Menge 2-Chlorbenzoxazol der Formel I vorzulegen, ist normalerweise nicht erforderlich. Das 2-Chlorbenzoxazol der Formel I wird zweckmäßigerweise in Form eines rohen Reaktionsgemischs eines vorhergehenden Ansatzes vorgelegt. Es schadet auch nichts, wenn sich in dem Reaktionsgefäß noch die Destillationsrückstände aus einem oder mehreren vorhergehenden Ansätzen befinden.

Das 2-Mercaptobenzoxazol der Formel II wird zweckmäßigerweise portionsweise oder kontinuierlich über einen Zeitraum von 0,5 bis 5 Stunden, vorzugsweise 1 bis 3 Stunden, eingetragen. Die Geschwindigkeiten der Chloreinleitung und der Zugabe des 2-Mercaptobenzoxazols der Formel II werden so aufeinander abgestimmt, daß das Reaktongsgemisch immer rührbar bleibt. Die Reaktion wird, falls eine Schmelze von reinem 2-Chlorbenzoxazol der Formel I vorgelegt wird, bei Temperaturen oberhalb des Schmelzpunkts des Endprodukts der Formel I, z. B. bei Temperaturen bis 100 °C, begonnen. Im Verlaufe der Reaktion kann die Reaktionstemperatur bis auf 0 °C abgesenkt werden.

In vielen Fällen wird die Reaktion im Temperaturbereich von 60 bis 10 °C durchgeführt. Vorzugsweise wird die Reaktion im Temperaturbereich von 40 bis 20 °C durchgeführt, insbesondere falls das 2-Chlorbenzoxazol der Formel I in Form eines rohen Reaktionsgemischs vorgelegt wird und/oder falls die Gefahr von Kernchlorierungen besteht. In jedem Fall muß die Temperatur jedoch so hoch gewählt werden, daß das Reaktionsgemisch noch rührbar ist. Nachdem die gesamte Menge 2-Mercaptobenzoxazol der Formel II eingetragen ist, wird mindestens noch so lange Chlor in das Reaktionsgemisch eingeleitet, bis die nach der Reaktionsgleichung II + $Cl_2$ → I + HCl + S berechnete Chlormenge verbraucht worden ist. Eine bessere Ausbeute erhält man, wenn so lange Chlor eingeleitet wird, bis die nach der Reaktionsgleichung 2 II + $3Cl_2$ → 2 I + 2HCl + $S_2Cl_2$ berechnete Menge verbraucht worden ist. Es kann zweckmäßig sein, die Einleitung von Chlor nach Erreichen der so berechneten Menge sofort abzubrechen, um unerwünschte Kernchlorierungen zu vermeiden. Im allgemeinen schadet es aber nichts, noch mehr Chlor einzuleiten. Es entsteht dann im Extremfall $SCl_2$ anstelle von $S_2Cl_2$, bzw. Gemische von $SCl_2$ und $S_2Cl_2$ entsprechend der Reaktionsgleichung II + $2Cl_2$ → I + HCl + $SCl_2$. Bevorzugt werden deswegen pro mol 2-Mercaptobenzoxazol der Formel II 1,5 mol Chlor eingesetzt. Die Aufarbeitung des Reaktionsgemisches erfolgt in üblicher Weise durch Destillation unter vermindertem Druck.

Nach dem erfindungsgemäßen Verfahren werden die 2-Chlorbenzoxazole der Formel I in ausgezeichneten Ausbeuten und hohen Reinheiten erhalten, und die gleichzeitig gebildeten Schwefelchloride $S_2Cl_2$ und/oder $SCl_2$ werden in hoher Reinheit ohne Verunreinigung durch organische Lösungsmittel gewonnen. Die Raumausbeute der Reaktion ist sehr hoch, und der Energiebedarf ist gering, da kein Lösungsmittel destilliert werden muß. Die Reinigung des Reaktionsgefäßes braucht nicht nach jedem Ansatz zu erfolgen, da der Destillationsrückstand ohne Verminderung von Ausbeute und Produktqualität mehrere Ansätze lang im Reaktionsgefäß verbleiben kann. Die als Ausgangsverbindungen benötigten 2-Mercaptobenzoxazole der Formel II lassen sich in an sich bekannter Weise aus den entsprechenden ortho-Aminophenolen durch Umsetzung mit Natriumethylxanthat oder mit Schwefelkohlenstoff in Gegenwart einer Base herstellen.

## Beispiel 1

In einem 1-l-Vierhalskolben mit Rührer, Innenthermometer, Gaseinleitungsrohr und 30-cm-Kolonne mit Raschigringen werden 150 g reines 2,6-Dichlorbenzoxazol (Fp 49-50 °C) vorgelegt und im Wasserbad bei 60 °C geschmolzen. In die Schmelze werden 835 g 2-Mercapto-6-chlorbenzoxazol portionsweise im Verlauf von ca. 1 bis 3 Stunden eingetragen. Gleichzeitig wird durch das Gaseinleitungsrohr Chlor eingeleitet. Eintraggeschwindigkeit und Chlorstrom werden so aufeinander abgestimmt, daß der Kolbeninhalt immer rührbar bleibt. Durch Außenkühlung wird die Innentemperatur anfangs unter 60 °C gehalten, später bis auf Raumtemperatur abgesenkt. Wenn alles 2-Mercapto-6-chlor-benzoxazol eingetragen ist, wird weiter Chlor eingeleitet, bis 480 g aufgenommen sind. Gesamtdauer der Chloreinleitung ca. 3 Stunden. Das Reaktionsgemisch wird im Wasserstrahlvakuum destilliert, wobei zunächst bei ca. 30 °C $S_2Cl_2$, dann bei 118 °C und 24 mbar ca. 920 g 2,6-Dichlorbenzoxazol übergehen.

Gaschromatographische Reinheit 99,8 %, Fp. 49 bis 50 °C,
Ausbeute 91 % der Theorie.
Analyse des gewonnenen $S_2Cl_2$ : 47,2 % S, 52,9 % Cl (theoretisch 47,5 % S, 52,5 % Cl).

## Beispiel 2

Verfährt man wie im Beispiel 1, legt jedoch nicht 2,6-Dichlorbenzoxazol vor, sondern ein die entsprechende Menge 2,6-Dichlorbenzoxazol enthaltendes rohes undestilliertes Reaktionsgemisch wie es im Beispiel 1 anfällt und hält während der gesamten Reaktion eine Temperatur von 30-40 °C ein, so erhält man ca. 940 g 2,6-Dichlorbenzoxazol von gleicher Reinheit. Ausbeute : 93 % der Theorie.

## Beispiel 3

Verfährt man wie in den Beispielen 1 und 2, leitet jedoch statt 480 g 600 g Chlor ein, so erhält man 2,6-Dichlorbenzoxazol in gleicher Ausbeute und Reinheit. Daneben fällt jedoch in diesem Falle nicht reines $S_2Cl_2$, sondern ein Gemisch von $SCl_2$ und $S_2Cl_2$ an.

## Beispiel 4

Verfährt man wie in den Beispielen 1 bis 3, benutzt jedoch einen Kolben, in dem sich noch die Destillationsrückstände von vorhergehenden Versuchen befinden, so erhält man 2,6-Dichlorbenzoxazol in gleicher Ausbeute und Reinheit.

## Beispiel 5

Verfährt man wie im Beispiel 1, jedoch mit dem Unterschied, daß 2-Chlorbenzoxazol vorgelegt und von Anfang an bei 20-40 °C gearbeitet wird, 830 g 2-Mercaptobenzoxazol eingetragen und 586 g Chlor

eingeleitet werden, so erhält man nach Destillation im Wasserstrahlvakuum bei 107 °C ca. 910 g 2-Chlorbenzoxazol. Im Destillationsrückstand läßt sich in Spuren kernchloriertes Produkt nachweisen. Ausbeute 90 % der Theorie.

Beispiel 6

Verfährt man wie im Beispiel 5, legt jedoch 5-Methyl-2-chlorbenzoxazol vor, trägt 825 g 5-Methyl-2-mercaptobenzoxazol ein und leitet 533 g Chlor ein, so erhält man nach Destillation im Wasserstrahlvakuum bei 100 °C ca. 830 g 5-Methyl-2-chlorbenzoxazol ; Ausbeute 81 % der Theorie. In diesem Falle muß besonders darauf geachtet werden, daß während der gesamten Reaktion die Temperatur von 40 °C nicht überschritten wird, da in diesem Fall eine erhöhte Gefahr der Kernchlorierung besteht.

**Patentansprüche**

1. Verfahren zur Herstelung von 2-Chlorbenzoxazolen der Formel I

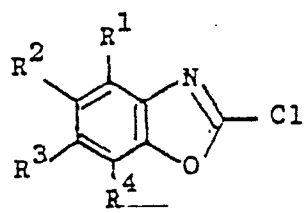

(I)

worin $R^1$, $R^2$, $R^3$, $R^4$ unabhängig voneinander H, Cl oder Alkyl mit 1 bis 4 C-Atomen bedeuten, durch Umsetzung von 2-Mercaptobenzoxazolen der Formel II

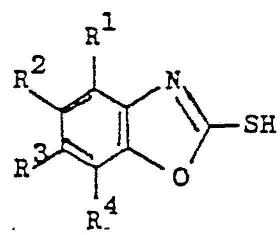

(II)

mit Chlor, dadurch gekennzeichnet, daß in ein geschmolzenes 2-Chlorbenzoxazol der Formel I 2-Mercaptobenzoxazol der Formel II, wobei die beiden Verbindungen der Formeln I und II in den Resten $R^1$ bis $R^4$ gleich substituiert sind, eingetragen und anschließend oder gleichzeitig Chlor eingeleitet wird und daß, bezogen auf die Gesamtmenge des eingetragenen 2-Mercaptobenzoxazols, die Menge des geschmolzenen 2-Chlorbenzoxazols mindestens 8 Gew.% beträgt.

2. Verfahren nach Anspruch 1, dadurch gekennzeichnet, daß als geschmolzene Verbindung der Formel I rohes Reaktionsgemisch eines vorhergehenden Ansatzes verwendet wird.

3. Verfahren nach Anspruch 1 oder 2, dadurch gekennzeichnet, daß das 2-Mercaptobenzoxazol portionsweise eingetragen wird.

4. Verfahren nach Anspruch 1 oder 2, dadurch gekennzeichnet, daß das 2-Mercaptobenzoxazol kontinuierlich eingetragen wird.

5. Verfahren nach einem oder mehreren der Ansprüche 1 bis 4, dadurch gekennzeichnet, daß das Chlor in dem Maße eingeleitet wird, daß das Reaktionsgemisch rührbar bleibt.

6. Verfahren nach einem oder mehreren der Ansprüche 1 bis 5, dadurch gekennzeichnet, daß pro mol 2-Mercaptobenzoxazol der Formel II 1 bis 2 mol, vorzugsweise 1,5 mol, Chlor eingeleitet werden.

7. Verfahren nach einem oder mehreren der Ansprüche 1 bis 6, dadurch gekennzeichnet, daß die Reaktion bei Temperaturen von 100° bis 0 °C, vorzugsweise bei Temperaturen von 40 bis 20 °C, durchgeführt wird.

8. Verfahren nach einem oder mehreren der Ansprüche 1 bis 7, dadurch gekennzeichnet, daß bezogen auf die Gesamtmenge des eingetragenen 2-Mercaptobenzoxazols, die Menge des geschmolzenen 2-Chlorbenzoxazols 8 bis 30 Gew.%, vorzugsweise 10 bis 25 Gew.%, beträgt.

9. Verfahren nach einem oder mehreren der Ansprüche 1 bis 8, dadurch gekennzeichnet, daß Verbindungen der Formeln I und II eingesetzt werden, bei denen einer der Reste $R^1$, $R^2$, $R^3$ oder $R^4$ Chlor und die anderen drei Reste H bedeuten.

10. Verfahren nach einem oder mehreren der Ansprüche 1 bis 9, dadurch gekennzeichnet, daß Verbindungen der Formeln I und II eingesetzt werden, bei denen $R^1=R^2=R^3=R^4=H$ bedeuten.

**Claims**

1. Process for preparing 2-chlorobenzoxazoles of the formula I

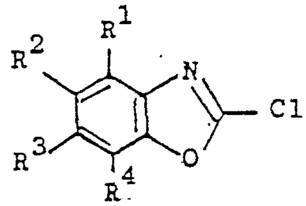

(I)

in which $R^1$, $R^2$, $R^3$ und $R^4$ independently of one another denote H, Cl or alkyl having 1 to 4 C atoms by reacting 2-mercaptobenzoxazoles of the formula II

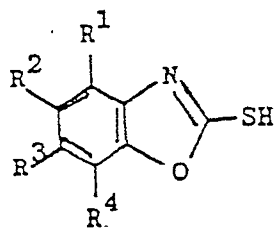

(II)

with chlorine, characterised in that 2-mercaptobenzoxazole of the formula II is added to a melted 2-chlorobenzoxazole of the formula I where the two compounds of the formulae I and II are identically substituted in the radicals $R^1$ to $R^4$, and chlorine is passed in afterwards or at the same time, and that, relative to the total amount of the melted 2-chlorobenzoxazole is at least 8 % by weight.

2. Process according to Claim 1, characterised in that crude reaction mixture of a previous batch is used as the melted compound of the formula I.

3. Process according to Claim 1 or 2, characterised in that the 2-mercaptobenzoxazole is added in portions.

4. Process according to Claim 1 or 2, characterised in that the 2-mercaptobenzoxazole is added continuously.

5. Process according to one or more of Claims 1 to 4, characterised in that the chlorine is passed in at such a rate that the reaction mixture remains stirrable.

6. Process according to one or more of Claims 1 to 5, characterised in that 1 to 2 mols, preferably 1.5 mols, of chlorine are passed in per mol of 2-mercaptobenzoxazole of the formula II.

7. Process according to one or more of Claims 1 to 6, characterised in that the reaction is carried out at temperatures of 100° to 0 °C, preferably at temperatures of 40 to 20 °C.

8. Process according to one or more of Claims 1 to 7, characterised in that, relative to the total amount of the 2-mercaptobenzoxazole is 8 to 30 % by weight, preferably 10 to 25 % by weight.

9. Process according to one or more of Claims 1 to 8, characterised in that compounds of the formulae I and II are used in which one of the radicals $R^1$, $R^2$, $R^3$ or $R^4$ denotes chlorine and the other three radicals denote H.

10. Process according to one or more of Claims 1 to 9, characterised in that compounds of the formulae I and II are used in which $R^1 = R^2 = R^3 = R^4 = H$.

**Revendications**

1. Procédé de préparation de 2-chlorobenzoxazoles de formule I ci-dessous

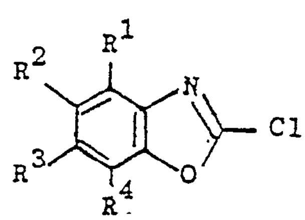

(I)

dans laquelle les symboles $R^1$, $R^2$, $R^3$ et $R^4$ désignent chacun, indépendamment les uns des autres, H, Cl ou un alkyle en $C_1$ à $C_4$, par réaction de 2-mercaptobenzoxazoles de formule II

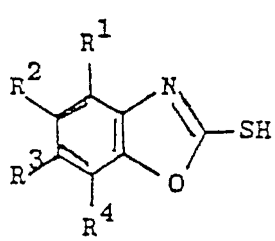

(II)

avec du chlore, procédé caractérisé en ce que l'on ajoute un 2-mercaptobenzoxazole de formule II à un 2-chlorobenzoxazole de formule I à l'état fondu, les symboles $R^1$ à $R^4$ ayant les mêmes significations pour ces deux composés, et, en même temps ou ensuite, on fait passer du chlore dans le mélange, et en ce que la proportion du 2-chlorobenzoxazole fondu est d'au moins 8 % du poids total du 2-mercaptobenzoxazole ajouté.

2. Procédé selon la revendication 1, caractérisé en ce que l'on part, comme composé fondu de formule I, du mélange réactionnel brut d'une opération précédente.

3. Procédé selon la revendication 1 ou 2, caractérisé en ce que l'on ajoute le 2-mercaptobenzoxazole par portions successives.

4. Procédé selon la revendication 1 ou 2, caractérisé en ce que l'on ajoute en continu le 2-mercaptobenzoxazole.

5. Procédé selon une ou plusieurs des revendications 1 à 4, caractérisé en ce que l'on fait passer le chlore à un débit tel que le mélange réactionnel demeure agitable.

6. Procédé selon l'une ou plusieurs des revendications 1 à 5, caractérisé en ce que l'on fait passer 1 à 2 moles de chlore, de préférence 1,5 mole, par mole de 2-mercaptobenzoxazole de formule II.

7. Procédé selon l'une ou plusieurs des revendications 1 à 6, caractérisé en ce que l'on effectue la réaction à des températures allant de 100 jusqu'à 0 °C, de préférence de 40 à 20 °C.

8. Procédé selon l'une ou plusieurs des revendications 1 à 7, caractérisé en ce que la proportion du 2-chlorobenzoxazole fondu est de 8 à 30 %, de préférence de 10 à 25 %, du poids total du 2-mercaptobenzoxazole ajouté.

9. Procédé selon l'une ou plusieurs des revendications 1 à 8, caractérisé en ce que l'on utilise des composés de formules I et II dans lesquelles, parmi $R^1$, $R^2$, $R^3$ et $R^4$, l'un est le chlore et les trois autres sont l'hydrogène.

10. Procédé selon l'une ou plusieurs des revendications 1 à 9, caractérisé en ce que l'on utilise des composés de formules I et II dans lesquelles $R^1$, $R^2$, $R^3$ et $R^4$ sont tous l'hydrogène.